Europäisches Patentamt

European Patent Office

Office européen des brevets

(11) Publication number: **0 282 989 B1**

# EUROPEAN PATENT SPECIFICATION

(45) Date of publication of patent specification: **06.10.93**

(51) Int. Cl.⁵: **C12P  19/32**, C12N 1/20,
//(C12N1/20,C12R1:425),
(C12P19/32,C12R1:425)

(21) Application number: **88104171.9**

(22) Date of filing: **16.03.88**

(54) **Process for producing 5'-inosinic acid.**

(30) Priority: **18.03.87 JP 63385/87**

(43) Date of publication of application:
**21.09.88 Bulletin  88/38**

(45) Publication of the grant of the patent:
**06.10.93 Bulletin  93/40**

(84) Designated Contracting States:
**DE FR GB**

(56) References cited:
CH-A- 432 528
GB-A- 1 015 556
GB-A- 1 118 995
GB-A- 1 145 310
US-A- 3 152 966

Chemical Abstracts, vol. 94, no. 7, Febr.
1981, p. 438, abstract 45603f, Columbus,
Ohio, US

(73) Proprietor: **KYOWA HAKKO KOGYO CO., LTD.**
**6-1, Ohte-Machi Itchome**
**Chiyoda-ku Tokyo(JP)**

(72) Inventor: **Fujio, Tatsuro**
**6-29-1, Sagamidai**
**Sagamihara-shi Kanagawa-ken(JP)**
Inventor: **Takeichi, Yasutoshi**
**4845-4, Ami**
**Ami-machi**
**Inashiki-gun Ibaraki-ken(JP)**
Inventor: **Kitatsuji, Katsura**
**Popuragaoka Coopu 14-206**
**2-9-5, Naruse**
**Machida-shi Tokyo(JP)**
Inventor: **Iida, Akihiro**
**4-17-9, Morino**
**Machida-shi Tokyo(JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing 5′-inosinic acid (which is hereinafter referred to as "IMP") by phosphorylating inosine by enzymatic reaction. IMP is used as a seasoning agent, and thus the present invention pertains to the field of food industry.

So far well known processes for producing IMP include (1) a process by enzymatically decomposing ribonucleic acid extracted from yeast cells (Japanese Published Examined Patent Application Nos. 16141/1957, 23698/1957, 7532/1958 and 35914/1958 and Food Technology 29, 287, (1964), (2) a process by chemically phosphorylating inosine produced by fermentation (Agric. Biol. Chem., 36, 1511 (1972) and Tetrahedron Letters, No. 50, 5065-5068 (1967) and (3) a process by culturing a microorganism capable of producing IMP and recovering the IMP accumulated in the medium (Agric. Biol. Chem., 46, 2257 (1982), etc. All of these processes are commercially utilized.

IMP is used not only in the ordinary home cooking as a seasoning agent, but also widely in the processed food such as boiled fish pastes, tubular rolls of boiled fish paste, instant noodles, various kinds of soup, etc. and the development of a process for producing IMP at lower cost has been desired.

The reaction for enzymatically phosphorylating inosine into IMP, using inosine and adenosine-5′-triphosphate (which is hereinafter referred to as ATP) as the substrate is catalyzed by an activity corresponding to inosine kinase (EC 2.7.1.73) to form IMP and an ATP precursor concurrently as illustrated by the following equation.

Inosine + ATP → IMP + ATP precursor

In an economically practical process for producing IMP, it is necessary to supply ATP at lower cost. In the phosphorylating reaction of inosine, the terminal phosphate group of ATP is taken into inosine to form IMP, while the ATP precursor portion remains as such. If there is a reaction system in which the energy necessary for synthesis of ATP is provided from an appropriate substrate and ATP is biosynthesized from the ATP precursor using the energy (the reaction system is hereinafter sometimes referred to as ATP regeneration system), it may be possible to establish an IMP formation system capable of repeatedly using the portion corresponding to the ATP precursor, that is, an IMP production system without supplying any ATP, by coupling the ATP regeneration system with the phosphorylation reaction of inosine as illustrated by the following equations. In that case, if the phosphate donor and the energy donor are cheap, an economically advantageous process for producing IMP can be established.

Inosine + ATP → IMP + ATP precursor

ATP precursor + phosphate donor + energy donor → ATP

The present inventors have found that IMP can be practically produced by using carbohydrates such as glucose as the main raw material for the inosine production, and further using a cheap energy donor and a cheap phosphate donor for regenerating ATP in place of ATP, when an action of an inosine-producing microorganism capable of directly producing inosine from a carbon source such as carbohydrates, etc. by fermentation (the microorganism is hereinafter sometimes referred to as inosine microorganism), an action of a microorganism having an activity to biosynthesize ATP from an ATP precursor, an energy donor and a phosphate donor (the microorganism is hereinafter sometimes referred to as ATP-regenerating microorganism and the activity is hereinafter sometimes referred to as ATP - regenerating activity) and an action of a microorganism capable of synthesizing IMP and an ATP precursor from inosine and ATP (the microorganism is hereinafter sometimes referred to as phosphorylating microorganism) are coupled, and further that the ATP-regenerating activity concurrently possessed by the inosine microorganism can be utilized in the ATP regeneration system, and thus the present invention has been established.

The present invention relates to a process for producing IMP, which comprises bringing inosine or an inosine-containing culture broth obtained by culturing a microorganism capable of producing inosine in a medium into contact with treated products of cells of a microorganism capable of biosynthesizing ATP from a precursor of ATP, an energy donor and a phosphate donor, and with treated products of cells of a microorganism capable of forming IMP and a precursor of ATP from inosine and ATP, in the presence of an energy donor and a phosphate donor, until IMP is accumulated in a reaction mixture and recovering IMP therefrom.

As the inosine or the inosine-containing culture broth, any of purified product or roughly purified product of inosine, a culture broth of inosine fermentation and a concentrate thereof, a supernatant free from the cells or a concentrate of the supernatant, etc. can be used, so long as it does not bar the phosphorylating reaction of inosine to IMP. A culture broth obtained by culturing the inosine microorganism is preferably used.

Any strain can be used as the inosine microorganism, so long as it can produce inosine in the medium. Specifically, Brevibacterium ammoniagenes ATCC 21295, Corynebacterium glutamicum ATCC 19185, Bacillus subtilis ATCC 14618, etc. are suitable.

A remarkable amount of inosine can be accumulated in the medium by culturing these microorganisms according to the ordinary culturing procedure. That is, these microorganisms are cultured in an ordinary medium containing appropriate carbon source, appropriate nitrogen source, appropriate inorganic materials, amino acids, vitamins, etc. under aerobic conditions while adjusting the temperature, pH, etc.

Culturing is carried out under aerobic conditions, for example, by shaking and by aeration and agitation, at a temperature of 20 to 40°C, preferably 25 to 35°C usually for 10 to 120 hours, while keeping the pH of the medium around neutral.

As the microorganism capable of biosynthesizing ATP from a precursor of ATP, an energy donor and a phosphate donor, any strain can be used, so long as it has an activity to regenerate ATP from a precursor of ATP, an energy donor and a phosphate donor. Particularly, Escherichia coli ATCC 33525, Escherichia coli ATCC 11303, Staphylococcus aureus ATCC 4012 Saccharomyces sake ATCC 20018, Candida zeylanoides ATCC 20356, Torulopsis psychrophila ATCC 22163, etc. can be used, besides the above-mentioned inosine microorganisms.

As the cells having an ATP-regenerating activity, a culture obtained by culturing these microorganisms according to the ordinary culturing procedure may be used as it is. Moreover, if desired, cells obtained by centrifuging the culture may also be used. That is, these microorganisms are cultured in an ordinary medium containing appropriate carbon source, appropriate nitrogen source, appropriate inorganic materials, amino acids, vitamins, etc. under aerobic conditions, while adjusting the temperature, pH, etc.

Culturing is carried out under aerobic conditions, for example, by shaking and by aeration and agitation, at a temperature of 20 to 50°C, preferably 25 to 43°C, usually for 10 to 120 hours, while keeping the pH of the medium around neutral. Centrifugation is carried out at 5,000 - 10,000 rpm for 10 - 20 minutes.

As the energy donor, any of carbohydrates such as glucose, arabinose, lactose, maltose, sucrose, mannitol, sorbitol, trehalose, molasses, blackstrap molasses, starch hydrolyzate, etc.; organic acids such as pyruvic acid, lactic acid, acetic acid, $\alpha$-ketoglutaric acid, etc.; and amino acids such as glycine, alanine, aspartic acid, glutamic acid, glutamine, etc. can be used, so long as it can be utilized by the ATP-regenerating microorganisms to be used. Furthermore, phosphorilated compounds such as acetylphosphoric acid, carbamylphosphoric acid, creatine phosphoric acid, etc. can be used. It is desirable to keep its concentration in a range of 10 g/l to 500 g/l.

As the phosphate donor, any of sodium salts, potassium salts, magnesium salts, etc. of inorganic phosphoric acids such as orthophosphoric acid, pyrophosphoric acid, polyphosphoric acid, polymetaphosphoric acid can be used. Furthermore, organic phosphorilated compounds such as acetylphosphoric acid, carbamylphosphoric acid, creatine phosphoric acid, fructose-1, 6-bisphosphoric acid, etc. can also be used. It is desirable to keep its concentration in a range of 10 to 400 mM.

As the microorganism capable of forming IMP and a precursor of ATP from inosine and ATP, any microorganism can be used, so long as it has an inosine-phosphorylating activity of forming IMP from inosine and ATP. Preferred microorganisms are Escherichia coli ATCC 14948, Escherichia coli ATCC 11303, Bacillus subtilis ATCC 14617, Flavobacterium devorans ATCC 10829. Serratia marcescens YT101 FERM BP-1291 is particularly preferred. Furthermore, strains derived from these microorganisms, whose inosine-phosphorylating activity is increased by a molecular breeding procedure such as genetic recombination, cell fusion, etc. are suitable.

As the cells having an activity of forming IMP from inosine and ATP, a culture obtained by culturing these microorganisms according to the ordinary culturing procedure may be used as it is. Moreover, if desired, cells obtained by centrifuging the culture may also be used. That is, these microorganisms are cultured in an ordinary medium containing appropriate carbon source, appropriate nitrogen source, appropriate inorganic materials, amino acids, vitamins, etc. under aerobic conditions, while adjusting the temperature, pH, etc.

Culturing is carried out under aerobic conditions, for example, by shaking and by aeration and agitation, at a temperature of 20 to 50°C, preferably 28 to 43°C, usually for 1 to 48 hours. It is desirable to keep the pH of the medium around neutral during the culturing. Centrifugation is carried out at 5,000 - 10,000 rpm for 10 - 20 minutes.

The carbon source for use in the culturing of inosine microorganisms, ATP-regenerating microorganisms and phosphorylating microorganisms includes carbohydrates such as glucose, fructose, sucrose, maltose, mannitol, sorbitol, etc.; various alcohols and organic acids such as sugar alcohol, glycerol, pyruvic acid, lactic acid, citric acid, etc.; and various amino acids such as glutamic acid, methionine, lysine, etc. Natural organic nutrient sources such as starch hydrolyzate, molasses, blackstrap molasses, white rice bran, cassava, bagasse, corn steep liquor, etc. can be used, so long as they can be assimilated by the microorganisms.

3

The nitrogen source includes ammonia; various inorganic and organic ammonium salts such as ammonium chloride, ammonium sulfate, ammonium carbonate, ammonium acetate, etc.; various amino acids such as glutamic acid, glutamine, methionine, etc.; and nitrogen-containing organic materials such as peptone, NZ amine, corn steep liquor, meat extract, yeast extract, casein hydrolyzate, fish meal or its digested product, chrysalis hydrolyzate, etc.

As the inorganic materials, potassium dihydrogen phosphate, sodium monohydrogen phosphate, magnesium sulfate, sodium chloride, calcium chloride, iron chloride, copper sulfate, manganese chloride, ammonium molybdate, zinc sulfate, etc. are added to the medium, if necessary. Vitamins, amino acids, minerals, nucleic acids and other materials necessary for the growth of the microorganisms are added to the medium, and if they are naturally supplied by other medium components described above, it is not necessary to add these specific nutrients separately to the medium.

The treated products of cells of ATP-regenerating microorganisms and phosphorylating microorganisms include dried residues, freeze-thawed products and freeze dried products of the cultures; surfactant- and/or organic solvent-treated culture, acetone-treated culture and bacteriolytic enzyme-treated culture; frozen cells, dried cells, freeze-dried cells, freeze-thawed cells, acetone-treated cells, surfactant- and/or organic solvent-treated cells and bacteriolytic enzyme-treated cells, immobilized cells thereof, etc. Furthermore, liquids containing ATP-regenerating enzyme or inosine-phosphorylating enzyme extracted from the cells, purified preparates and immobilized products of these enzymes, etc. are appropriate for this purpose.

By contacting the inosine or inosine-containing culture broth, the treated cells of ATP-regenerating microorganism, and the treated cells of phosphorylating microorganism with a phosphate donor and an energy donor, IMP is accumulated in the reaction mixture. If necessary, an ATP precursor may be added in a reaction mixture.

Phosphorylation of inosine to IMP is carried out by adding magnesium ions, a surfactant and/or an organic solvent to the reaction mixture, if necessary, while adjusting the pH to 6 - 10, preferably 7 - 8 and keeping the reaction mixture at 20 to 50°C for 1 to 48 hours. It is desirable that the concentration of inosine is in a range of 1 to 100 mg/mℓ in the phosphorylating reaction of inosine to IMP.

As the ATP precursor, any of purified preparates and roughly purified preparates of adenosine 5'-diphosphate, adenosine 5'-monophosphate, adenosine, adenine, etc., and materials containing these compounds can be used, so long as it does not bar the phosphorylating reaction of inosine to IMP. If the amount of the ATP precursor to be brought into the reaction system from the cells or culture is sufficient, its addition is not necessary.

As the surfactant , any of cationic surfactants such as polyoxyethylene stearylamine (for example, Nymeen S-215, product of Nihon Oil and Fats Co., Ltd., Japan), cetyltrimethylammonium bromide, Cation FB, Cation F2-40E (product of Nihon Oil and Fats Co., Ltd., Japan), etc.; anionic surfactants such as sodium oleylamide sulfate, Nyurekkusu TAB, Rapizoru 80 (product of Nihon Oil and Fats Co., Ltd., Japan), etc.; amphoteric surfactants such as polyoxyethylenesorbitan monostearate (for example, Nonion ST 221, product of Nihon Oil and Fats Co., Ltd., Japan), etc.; and Sankyu amine PB (product of Nihon Oil and Fats Co., Ltd., Japan), hexadecyldimethylamine, etc. can be used, so long as it can promote phosphorylation of inosine to IMP. The surfactant is used at a concentration of usually, 0.1 to 50 mg/mℓ, preferably 1 to 20 mg/mℓ. Furthermore, as the organic solvent, toluene, xylene, aliphatic alcohols, benzene, ethyl acetate, etc. can be used at a concentration of 0.1 to 50 μℓ/mℓ, preferably 1 to 20 μℓ/mℓ.

It is desirable to keep a concentration of magnesium ions in a range of 4 to 400 mM in the phosphorylating reaction of inosine to IMP. When the amount of magnesium ions to be brought into the phosphorylation system from the culture or cells falls within this concentration range it is not necessary to add these ions, whereas, when these ions are short, they are added thereto so that the amount may fall within this concentration range. As the magnesium ion, any of inorganic and organic magnesium salts can be used.

Recovery of IMP from the reaction mixture can be carried out according to an ordinary procedure using activated carbon, ion exchange resin, etc.

The present invention is described below, referring to Examples.

Example 1

One loopful of Brevibacterium ammoniagenes ATCC 21295 was inoculated in a 50 mℓ-large test tube containing 10 mℓ of a seed medium (pH 7.2) comprising 1% polypeptone, 0.5% meat extract, 0.5% yeast extract and 0.25% sodium chloride, and cultured by reciprocative shaking at 30°C for 24 hours. Then, 2 mℓ of the seed culture was each inoculated in 300 mℓ-Erlenmeyer flasks provided with baffles each containing 20 mℓ of a medium comprising 15% glucose, 0.01% casein hydrolyzate, 0.7% yeast extract, 1.0% ammonium sulfate, 0.3% $KH_2PO_4$, 0.3% $K_2HPO_4$, 0.5% $MgSO_4 \cdot 7H_2O$, $10\mu g/m\ell$ each of adenine and guanine and 10 $\mu g/\ell$ of biotin, adjusted to pH 7.2 and autoclaved at 120°C for 20 minutes. During the culture by rotary shaking at 30°C, the pH was kept around neutral by adding urea to the medium, if necessary, and 21.1 mg/m$\ell$ inosine was formed in the medium by culturing for 72 hours. A supernatant free from cells of inosine microorganism by centrifuge was obtained.

Each of three strains, i.e Saccharomyces sake ATCC 20018, Torulopsis psychrophila ATCC 22163 and Candida zeylanoides ATCC 20356 was inoculated into a 2$\ell$-Erlenmeyer flask containing 300 m$\ell$ of a seed culture medium comprising 30 g/$\ell$ glucose, 5 g/$\ell$ ammonium sulfate, 1 g/$\ell$ $KH_2PO_4$, 0.5 g/$\ell$ $MgSO_4 \cdot 7H_2O$, 3 g/$\ell$ yeast extract and 10 g/$\ell$ $CaCO_3$ (pH 6.5 before sterilization) and cultured at 30°C for 24 hours. Then, 30 m$\ell$ of the seed culture was inoculated in a 2 $\ell$-Erlenmeyer flask containing 300 m$\ell$ of a producing medium comprising 150 g/$\ell$ glucose, 10 g/$\ell$ ammonium sulfate, 1 g/$\ell$ $KH_2PO_4$, 0.5 g/$\ell$ $MgSO_4 \cdot 7H_2O$ and 5 g/$\ell$ corn steep liquor (pH 6.5 before sterilization) and cultured by rotary shaking at 30°C for 48 hours, while adjusting the pH to about 6.5 with ammonia during the culturing. Cells were recovered from the culture by centrifugation.

One loopful each of Escherichia coli ATCC 33525, Escherichia coli ATCC 11303, Staphylococcus aureus ATCC 4012, and Serratia marcescens YT 101 (FERM BP-1291) were inoculated in a large test tube containing 10 m$\ell$ of a seed medium (pH 7.2) having the same composition as above after the sterilization and cultured by reciprocative shaking at 30°C for 20 hours. Then, 2 m$\ell$ of the seed culture was inoculated in a 2$\ell$-Erlenmeyer flask containing 300 m$\ell$ of M9 medium (6 mg/m$\ell$ $Na_2HPO_4$, 3 mg/m$\ell$ $KH_2PO_4$, 5 mg/m$\ell$ NaCl, 1 mg/m$\ell$ $NH_4Cl$, 4 $\mu g/m\ell$ thiamine•HCl, 3 mg/m$\ell$ glucose and 0.25 mg/m$\ell$ $MgSO_4 \cdot 7H_2O$, pH 7.2) and cultured by rotary shaking at 30°C for 17 hours. Cells were recovered from the culture by centrifugation.

Saccharomyces sake ATCC 20018, Torulopsis phychrophila ATCC 22163, Candida zeylanoides ATCC 20356, Escherichia coli ATCC 33525, Escherichia coli ATCC 11303 or Staphylococcus aureus ATCC 4012 each having an ATP-regenerating activity was added to the supernatant of the inosine fermentation culture of Brevibacterium ammoniagenes ATCC 21295 to make the concentration of the strains to be 100 mg/m$\ell$ (wet cell weight) ,and also cells of Serratia marcescens YT 101 (FERM BP-1291) having an activity to phosphorylate inosine to form IMP was added thereto to make the concentration 100 mg/m$\ell$ (wet cell weight). To each the thus obtained respective cell suspensions were added 50 mg/m$\ell$ glucose, 8 mg/m$\ell$ of 25% sodium phytate (pH 7.0), 5 mg/m$\ell$ $Na_2HPO_4$ and 5 mg/m$\ell$ $MgSO_4 \cdot 7H_2O$, and further 4 mg/m$\ell$ Nymin S-215 and 10 $\mu\ell/m\ell$ xylene were added thereto (in the case of the genera Escherichia and Staphylococcus), and 1 g/$\ell$ Sankyu amine PB was added thereto (in the case of the genera Saccharomyces, Torulopsis and Candida), and 20 m$\ell$ of the thus obtained suspensions were put separately into 200 m$\ell$-beakers, and reaction was carried out at 30°C for 24 hours with stirring at 900 rpm with a magnetic stirrer while keeping the suspensions at a pH of about 7.4 with ammonia to conduct phosphorylating reaction of inosine to IMP. The results are shown in Table 1.

## Table 1

| Strain | | IMP* (mg/m$\ell$) |
|---|---|---|
| Saccharomyces sake | ATCC 20018 | 5.70 |
| Toluropsis phychrophila | ATCC 22163 | 4.01 |
| Candida zeylanoides | ATCC 20356 | 4.30 |
| Escherichia coli | ATCC 33525 | 5.56 |
| Escherichia coli | ATCC 11303 | 6.82 |
| Staphylococcus aureus | ATCC 4012 | 3.35 |

* In terms of IMP·$Na_2$·$7H_2O$; the same basis is hereinafter applied.

Example 2

One loopful of Brevibacterium ammoniagenes ATCC 21295 was inoculated in a 70 mℓ-large test tube containing 10 mℓ of a seed medium (pH 7.2) comprising 1% polypeptone, 0.5% meat extract, 0.5% yeast extract and 0.25% sodium chloride and cultured by reciprocative shaking at 30°C for 24 hours.

Separately, a medium comprising 15% glucose, 0.01% casein hydrolyzate, 0.7% yeast extract, 1.0% ammonium sulfate, 0.3% $KH_2PO_4$, 0.3% $K_2HPO_4$, 0.5% $MgSO_4 \cdot 7H_2O$, 10 μg/mℓ each of adenine and guanine and 10 μg/ℓ biotin was adjusted to pH 7.2 and 20 mℓ of the medium was put into a 300 mℓ-Erlenmeyer flask provided with baffles. The medium was autoclaved at 120°C for 20 minutes, and then 2 mℓ of the seed culture was inoculated therein, and culturing is carried out by rotary shaking at 30°C, while keeping the pH around neutral by adding urea thereto, if necessary. Then, 20.0 mg/mℓ inosine was formed in the medium by culturing for 76 hours.

One loopful of Serratia marcescens YT 101 (FERM BP-1291) was inoculated in a large test tube containing 10 mℓ of sterilized seed medium (pH 7.2) having the same composition as above and cultured by reciprocative shaking at 30°C for 20 hours, and then 2 mℓ of the thus obtained seed culture was inoculated in a 2ℓ-Erlenmeyer flask containing 300 mℓ of M9 medium and cultured by rotary shaking at 30°C for 17 hours. The cells were recovered from the culture by centrifugation and preserved by freezing at -20°C.

The frozen of Serratia marcescens YT 101 (FERM BP-1291) were suspended in water and added to inosine fermentation liquor to make the concentration 50 mg/mℓ as wet cell weight, and 50 mg/mℓ glucose, 8 mg/mℓ of 25% sodium phytate (pH 7.0), 5 mg/mℓ $Na_2HPO_4$ and 5 mg/mℓ $MgSO_4 \cdot 7H_2O$ were added thereto, and furthermore 4 mg/mℓ Nymeen S-215 and 10 μℓ/mℓ xylene were added thereto. Then, 20 mℓ of the suspension was put into a 200 mℓ-beaker, and subjected to phosphorylating reaction of inosine to IMP at 30°C for 24 hours with stirring at 900 rpm with a magnetic stirrer while adjusting the pH to about 7.4 with ammonia. It was found that 8.80 mg/mℓ IMP was formed and accumulated in the reaction mixture, when neither Nymeen S-215 nor xylene was added, 0.6 mg/mℓ IMP was accumulated. When the cells of Serratia marcescens were not added, the amount of accumulated IMP was less than 0.3 mg/mℓ.

Example 3

Culturing and reaction were carried out in the same manner as in Example 2 except that Corynebacterium glutamicum ATCC 19185 was used as the inosine microorganism. The amount of inosine formed was 7.35 mg/mℓ and that of IMP formed was 3.44 mg/mℓ.

Example 4

Culturing was carried out in the same manner as in Example 2. The amount of inosine formed was 18.9 mg/mℓ. Reaction was carried out in the same manner as in Example 2 by using (A) the culture obtained above and (B) a supernatant obtained from the inosine fermentation culture by centrifugation except that the strains shown in Table 2 were used in place of Serratia marcescens YT 101 (FERM BP-1291). The results are shown in Table 2.

## Table 2

| Strain | | IMP (mg/mℓ) | |
|---|---|---|---|
| | | (A) | (B) |
| Escherichia coli | ATCC 39023 | 3.21 | 0.22 |
| Escherichia coli | ATCC 11303 | 2.33 | 0.18 |
| Bacillus subtilis | ATCC 14617 | 2.29 | 0.21 |
| Flavobacterium devorans | ATCC 10829 | 3.41 | 0.22 |

Example 5

Brevibacterium ammoniagenes ATCC 21295 was cultured for 74 hours in the same manner as in Example 2 and a fermentation culture containing 18.3 mg/mℓ inosine was obtained.

Separately, Serratia marcescens YT 101, FERM BP-1291 was cultured in the same manner as in Example 2 and the cells were recovered from the thus obtained culture by centrifugation. The cells were suspended in a phosphate buffer solution (pH 7.0) to make the concentration 200 mg/mℓ as wet cell weight and intermittently disintegrated in an ultrasonic disintegrator (type UR-200P, made by Tomy Seiko K.K., Japan) under ice cooling conditions totally for 10 minutes. Then, 100 mℓ of the cell-disintegrated suspension was centrifuged at 10,000 rpm for 10 minutes and the resulting supernatant was concentrated to 10 mℓ by a molecular sieve membrane (standard cell model 52 of Amicon Co., provided with Diaflow membrane YM10 of the same company). The concentrate product was used for reaction.

Then, 2 mℓ of the cell extraction concentrate of Serratia marcescens was added to 18 mℓ of inosine fermentation culture and 50 mg/mℓ glucose, 8 mg/mℓ of 25% sodium phytate (pH 7.0), 5 mg/mℓ Na$_2$HPO$_4$ and 5 mg/mℓ MgSO$_4$•7H$_2$O were added thereto, and furthermore 4 mg/ml Nymeen S-215 and 10 μl/ml xylene were added thereto. Then, 20 mℓ of the resulting mixture was put into a 200 mℓ-beaker and subjected to the reaction in the same manner as in Example 2. It was found that 6.44 mg/mℓ IMP was formed in the reaction mixture.

Example 6

Brevibacterium ammoniagenes ATCC 21295 was cultured in the same manner as in Example 2, whereby an inosine fermentation culture containing 19.3 mg/mℓ inosine and cells was obtained.

Separately, Serratia marcescens YT 101, FERM BP-1291 was cultured in the same manner as in Example 2 and the cells were recovered from the culture by centrifugation.

The cells of Serratia marcescens were suspended in (1) distilled water, (2) 0.4% Nymeen solution, (3) 1% xylene solution, or (4) 0.4% Nymeen -1% xylene solution, and each of the suspensions was added to the inosine fermentation culture to make 50 mg/mℓ as wet cell weight. Then, 50 mg/mℓ glucose, 8 mg/mℓ of 25% sodium phytate (pH 7.0), 5 mg/mℓ Na$_2$HPO$_4$ and 5 mg/mℓ MgSO$_4$•7H$_2$O were added thereto, and 20 mℓ each of the mixtures was put into 200 mℓ-beakers and subjected to IMP formation reaction in the same manner as in Example 2. The results are shown in Table 3.

Table 3

| Treating conditions | IMP (mg/mℓ) |
|---|---|
| No treatment | 0.3 |
| + N | 3.4 |
| + X | 5.4 |
| + N + X | 8.5 |
| N : 0.4% Nymeen S-215 | |
| X : 1% Xylene | |
| N + X : 0.4% Nymeen + 1% Xylene | |

**Claims**

1. A process for producing 5'-inosinic acid, which comprises bringing inosine or a culture broth obtained by culturing a microorganism belonging to the genus Brevibacterium, Corynebacterium or Bacillus and capable of producing inosine in a medium into contact with treated products of cells of a microorganism belonging to the genus Escherichia, Staphylococcus, Saccharomyces, Candida or Torulopsis and capable of biosynthesizing adenosine-5'-triphosphate from a precursor of adenosine-5'-triphosphate, an energy donor and a phosphate donor, and with treated products of cells of a microorganism belonging to the genus Escherichia, Bacillus, Flavobacterium or Serratia and capable of forming 5'-inosinic acid and a precursor of adenosine-5'-triphosphate from inosine and adenosine-5'-triphosphate, in the presence of an energy donor and a phosphate donor, until 5'-inosinic acid is accumulated in a reaction mixture and recovering 5'-inosinic acid therefrom and in which the treated products of cells are selected from the group consisting of dried residues, freeze-thawed products and freeze-dried products of cultures; surfactant- and/or organic solvent-treated cultures, acetone-treated cultures and bacteriolytic enzyme-treated cultures; and frozen cells, dried cells , freeze-dried cells, freeze-thawed cells, acetone-treated cells, surfactant- and/or organic solvent-treated cells and bacteriolytic enzyme-treated cells.

2. The process according to claim 1, wherein the microorganism capable of producing 5'-inosinic acid is the microorganism capable of biosynthesizing adenosine-5'-triphosphate from the precursor of adenosine-5'-triphosphate, the energy donor and the phosphate donor.

3. The process according to claim 1 or 2, wherein the energy donor is selected from the group consisting of carbohydrates, organic acids and amino acids.

4. The process according to any one of claims 1 to 3, wherein the phosphate donor is selected from the group consisting of inorganic phosphoric acid and salts thereof, and organic phosphorylated compounds.

5. Use of Serratia marcescens YT 101 FERM BP-1291 for carrying out the process according to any one of claims 1-4.

**Patentansprüche**

1. Verfahren zur Herstellung von 5'-Inosinsäure, umfassend das Inkontaktbringen von Inosin oder einer durch Züchtung eines Mikroorganismus, der zur Gattung Brevibacterium, Corynebacterium oder Bacillus gehört und Inosin in einem Medium herstellen kann, erhaltenen Kulturbrühe mit behandelten Produkten aus Zellen eines Mikroorganismus, der zur Gattung Escherichia, Staphylococcus, Saccharomyces, Candida oder Torulopsis gehört und Adenosin-5'-triphosphat aus einer Vorstufe von Adenosin-5'-triphosphat, einer Energiequelle sowie einem Phosphatdonor biosynthetisch herstellen kann, und mit behandelten Produkten aus Zellen eines Mikroorganismus, der zur Gattung Escherichia, Bacillus, Flavobacterium oder Serratia gehört, und 5'-Inosinsäure und eine Vorstufe von Adenosin-5'-triphosphat aus Inosin und Adenosin-5'-triphosphat in Gegenwart einer Energiequelle und eines Phosphatdonors erzeugen kann, bis 5'-Inosinsäure in einem Reaktionsgemisch angehäuft ist, und die Gewinnung von 5'-Inosinsäure daraus und wobei die behandelten Produkte aus Zellen ausgewählt sind, die aus getrockneten Resten, durch Einfrieren und Auftauen erhaltenen Produkten von Kulturen, gefriergetrockneten Produkten von Kulturen, aus mit grenzflächenaktiven Mitteln und/oder organischen Lösungsmitteln behandelten Kulturen, mit Aceton behandelten Kulturen, mit bakteriolytischen Enzymen behandelten Kulturen, aus gefrorenen Zellen, getrockneten Zellen, gefriergetrockneten Zellen, durch Einfrieren und Auftauen behandelten Zellen, mit Aceton behandelten Zellen, mit grenzflächenaktiven Mitteln und/oder organischen Lösungsmitteln behandelten Zellen oder mit bakteriolytischen Enzymen behandelten Zellen besteht.

2. Verfahren nach Anspruch 1, wobei der zur Herstellung von 5'-Inosinsäure fähige Mikroorganismus derjenige ist, der Adenosin-5'-triphosphat aus der Vorstufe von Adenosin-5'-triphosphat, der Energiequelle und dem Phosphatdonor biosynthetisch herstellen kann.

3. Verfahren nach Anspruch 1 oder 2, wobei die Energiequelle ausgewählt ist aus Kohlenhydraten, organischen Säuren oder Aminosäuren.

4. Verfahren nach einem der Ansprüche 1 bis 3, wobei der Phosphatdonor ausgewählt ist aus anorganischen Phosporsäuren und Salzen davon oder organischen phosphorylierten Verbindungen.

5. Verwendung von Serratia marcescens YT 101 FERM BP-1291 zur Durchführung des Verfahrens nach einem der Ansprüche 1 bis 4.

**Revendications**

1. Procédé de production de l'acide 5'-inosinique, qui comporte le fait de mettre de l'inosine, ou un bouillon de culture obtenu par culture d'un microorganisme appartenant au genre Brevibacterium, Corynebacterium ou Bacillus et capable de produire de l'inosine dans un milieu, en contact avec des produits de traitement de cellules d'un microorganisme appartenant au genre Escherichia, Staphylococcus, Saccharomyces, Candida ou Torulopsis et capable de biosynthétiser de l'adénosine-5'-triphosphate à partir d'un précurseur d'adénosine-5'-triphosphate, d'un donneur d'énergie et d'un donneur de phosphate, et avec des produits de traitement de cellules d'un microorganisme appartenant au genre Escherichia, Bacillus, Flavobacterium ou Serratia et capable de former de l'acide 5'-inosinique et un précurseur d'adénosine-5'-triphosphate à partir d'inosine et d'adénosine-5'-triphosphate, en présence d'un donneur d'énergie et d'un donneur de phosphate, jusqu'à ce que de l'acide 5'-inosinique se soit accumulé dans le mélange réactionnel, et le fait de récupérer l'acide 5'-inosinique à partir de ce mélange, procédé dans lequel les produits de traitement de cellules sont choisis dans le groupe constitué par des résidus séchés, des produits de congélation-décongélation et des produits de lyophilisation de cultures, des cultures traitées par un tensio-actif et/ou un solvant organique, des cultures traitées à l'acétone et des cultures traitées par une enzyme bactériolytique, et des cellules congelées, des cellules séchées, des cellules lyophilisées, des cellules congelées-décongelées, des cellules traitées à l'acétone, des cellules traitées par un tensio-actif et/ou un solvant organique et des cellules traitées par une enzyme bactériolytique.

2. Procédé conforme à la revendication 1, dans lequel le microorganisme capable de produire de l'acide 5'-inosinique est le microorganisme capable de biosynthétiser de l'adénosine-5'-triphosphate à partir du précurseur d'adénosine-5'-triphosphate, du donneur d'énergie et du donneur de phosphate.

3. Procédé conforme à la revendication 1 ou 2, dans lequel le donneur d'énergie est choisi dans le groupe constitué par les hydrates de carbone, les acides organiques et les acides aminés.

4. Procédé conforme à l'une quelconque des revendications 1 à 3, dans lequel le donneur de phosphate est choisi dans le groupe constitué par les acides phosphoriques inorganiques, leurs sels et les composés organiques phosphorylés.

5. Emploi de Serratia marcescens YT 101, FERM BP-1291, pour mettre en oeuvre un procédé conforme à l'une quelconque des revendications 1 à 4.